# FASCICULE DE BREVET EUROPEEN

(11) **EP 0 714 621 B1**
(45) Date de publication et mention de la délivrance du brevet: **01.09.1999**
(21) Numéro de dépôt: 95402675.3
(22) Date de dépôt: 28.11.1995
(51) Int. Cl.: A46B 3/18

(54) **Dispositif de nettoyage de conduits d'instruments médicaux**
Reinigungsvorrichtung für Rinnen von medizinischen Geräten
Cleaning device for ducts of medical instruments

(30) Priorité: 30.11.1994 FR 9414363
(43) Date de publication de la demande: 05.06.1996
(73) Titulaire: LA TECHNOLOGIE AVANCEE MEDICALE, 93100 Montreuil (FR)
(72) Inventeur: Bourrelly Julien, F-94600 Choisy-le-Roi (FR)
(74) Mandataire: Hasenrader, Hubert

(56) Documents cités:
- EP-A- 0 467 126
- US-A- 1 825 929
- US-A- 5 168 593

## Description

La présente invention concerne un dispositif de nettoyage de conduits d'instruments médicaux d'investigation ou de prélèvement interne, comprenant un élément cylindrique allongé ayant une surface externe lisse et une brosse, prévue à une première extrémité dudit élément, montée sur une tige longitudinale et présentant des poils sensiblement radiaux.

Par instruments médicaux d'investigation ou de prélèvement interne, on entend des instruments comme ceux qui sont habituellement utilisés pour les examens d'endoscopie, présentant un conduit qu'on introduit dans le corps du patient et dans lequel on insère des outils médicaux tels que des appareils optiques, des outils de prélèvement ou encore des outils chirurgicaux.

De tels instruments sont relativement coûteux et doivent donc pouvoir être utilisés plusieurs fois. Entre deux utilisations, ils doivent subir un nettoyage rigoureux, puis être décontaminés ou stérilisés. Le nettoyage de l'intérieur des conduits s'avère très délicat et nécessite l'utilisation, à la manière d'un écouvillon, d'un dispositif du type précité.

Ce dispositif doit présenter des dimensions radiales suffisamment faibles pour pouvoir s'insérer dans le conduit, dont le diamètre est en général petit, par exemple de l'ordre de 1 à 20 mm. Par ailleurs, le dispositif doit être suffisamment flexible pour pouvoir épouser les coudes éventuels du conduit et, notamment, pour pouvoir parfaitement nettoyer les zones de bifurcation. Malgré ses faibles dimensions radiales et sa flexibilité, le dispositif doit présenter une relative rigidité axiale, c'est-à-dire une tenue suffisante à la traction et à la poussée, afin de pouvoir être inséré, en étant poussé par une de ses extrémités, dans toute la longueur du conduit qui peut atteindre un ou deux mètres.

Le brevet US 5 168 593 montre un dispositif de nettoyage dans lequel la brosse est raccordée à l'élément cylindrique de support par l'intermédiaire d'un ressort hélicoïdal. Plus précisément, la tige de la brosse est fixée à une première extrémité du ressort, l'autre extrémité de ce dernier étant fixée à l'élément cylindrique allongé.

Ceci permet de rendre flexible la liaison entre la brosse et l'élément cylindrique.

Malheureusement, ces dispositions ne confèrent aucune flexibilité à la brosse en elle-même.

La tige de la brosse servant de support aux poils, il n'est pas possible de lui conférer seulement une flexibilité radiale, sans la rendre également axialement flexible.

Pour permettre à la brosse de passer dans des zones des conduits à forte courbure, une première solution consiste à la doter d'une tige flexible. Malheureusement, la flexibilité axiale est nuisible à l'introduction de la brosse dans les conduits et met directement en cause son efficacité. De toute façon, le simple fait de doter une brosse longue d'une tige flexible n'est pas satisfaisant dans la mesure où une telle tige n'est pas en elle-même assez solide et se casse après des flexions répétées.

Une deuxième solution consiste à réaliser une brosse très courte (de longueur sensiblement égale à son diamètre), dotée d'une tige rigide, en s'appuyant sur le fait qu'étant donné la faible longueur de la brosse, il n'est pas nécessaire que la tige se plie fortement, même dans des zones à fortes courbure. Une telle brosse présente l'inconvénient d'être trop courte pour correctement nettoyer certains conduits particulièrement sales.

Le fait de conférer au dispositif une souplesse locale dans la zone de liaison entre la brosse et l'élément cylindrique de support (lui-même suffisamment souple radialement et suffisamment rigide axialement) ne suffit donc pas à assurer la fiabilité du dispositif.

L'invention a pour but de remédier aux inconvénients précités.

Dans ce but, le dispositif comporte, en outre, un organe de maintien de la brosse, monté à la première extrémité de la fibre, de diamètre externe sensiblement égal à celui de cette fibre et susceptible de maintenir la brosse sur sensiblement toute la longueur de cette dernière, ledit organe étant constitué par un fil enroulé en hélice, dont les spires délimitent un canal apte à recevoir la tige de la brosse, et présentant un tronçon, de longueur sensiblement égale à celle de la brosse, sur lequel les spires sont espacées axialement de manière à permettre le passage des poils de la brosse entre ces spires.

Grâce à ces dispositions, l'invention permet d'utiliser une brosse longue, montée sur une tige flexible, sans que cette flexibilité ne nuise à l'efficacité du nettoyage ou à l'insertion de la brosse dans le conduit à nettoyer, cette brosse étant maintenue axialement par l'organe de maintien, sans que celui-ci ne s'oppose à la flexibilité radiale nécessaire au passage dans les zones à fortes courbures.

L'invention sera bien comprise et ses avantages apparaîtront mieux à la lecture de la description détaillée qui suit, de modes de réalisation représentés à titre d'exemples non limitatifs.

La description se réfère aux dessins annexés sur lesquels :

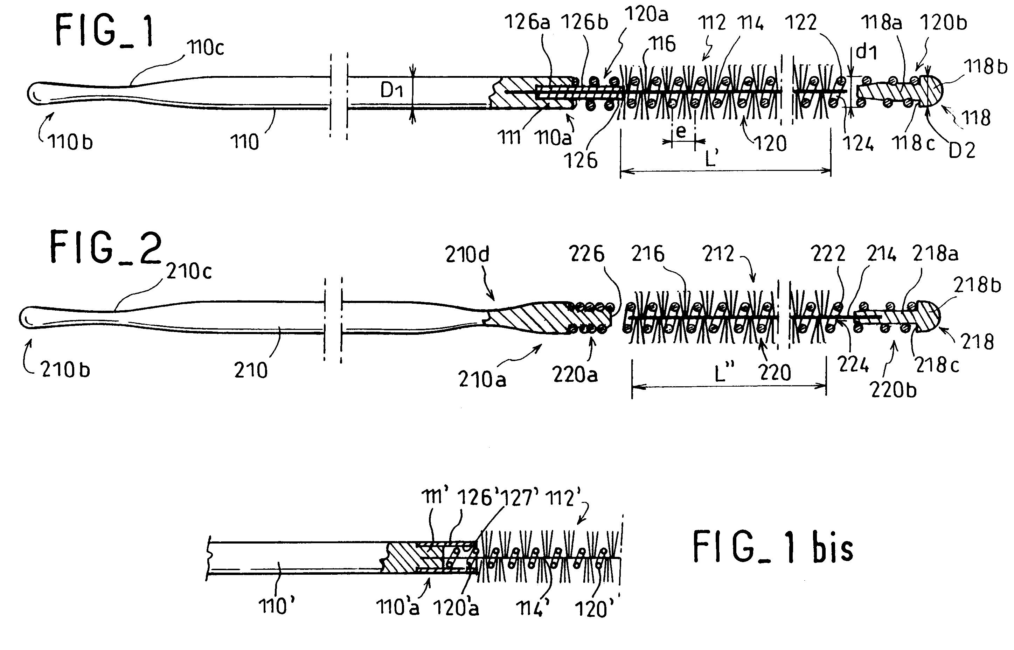

- la figure 1 montre en élévation latérale un premier mode de réalisation du dispositif selon l'invention,
- la figure 1bis montre un détail d'une variante du mode de réalisation de la figure 1,
- la figure 2 présente un deuxième mode de réalisation,
- les figure 3 à 7 sont des vues partielles illustrant des variantes de réalisation.

Le dispositif de la figure 1 comprend un élément cylindrique allongé 110 et une brosse 112, prévue à une première extrémité 110a de l'élément 110, montée sur une tige longitudinale 114 et pourvue de poils 116 sensiblement radiaux.

Pour alléger le dessin, on a tronqué l'élément cylindrique allongé, dont la longueur peut aller de un à plusieurs mètres, tandis que son diamètre est en général compris entre 1 et 20 mm. Dans l'exemple représenté, cet élément cylindrique allongé est constitué par une fibre lisse pleine réalisée en matière plastique (telle que Nylon, PVC ou Téflon), par exemple par extrusion. Il peut également être constitué par une fibre plastique armée. De manière générale, la surface externe de l'élément 110 est lisse. Cet élément peut donc être simplement revêtu par du plastique.

L'extrémité du dispositif située du côté de la brosse 112 est munie d'un embout de protection 118, par exemple en matière plastique, qui présente une extrémité libre 118b arrondie, de diamètre D2 sensiblement égal au diamètre courant D1 de la fibre 110.

Par diamètre courant de la fibre, on entend le diamètre de cette dernière sur la majeure partie de sa longueur, à l'exclusion d'éventuelles parties resserrées. En fait, son diamètre courant correspond également à son diamètre maximal.

Le dispositif comporte un organe de maintien de la brosse. En effet, pour augmenter l'efficacité, on a intérêt à doter la brosse d'une longueur relativement importante.

Comme on l'a indiqué précédemment, lorsque la brosse est longue, il faut qu'elle soit flexible pour pouvoir épouser les courbes des conduits d'instruments qu'elle nettoie. En fait, les mêmes exigences en matière de flexibilité radiale et de relative rigidité axiale s'appliquent à la brosse et au reste du dispositif. Une simple tige flexible longue ne satisfait pas à ces exigences et risque de se casser.

L'organe de maintien permet d'équiper le dispositif de brosses longues, dont la longueur est nettement supérieure au diamètre, par exemple comprise entre cinq et quinze fois le diamètre, voire davantage.

L'organe de maintien 120 de la figure 1 est monté à la première extrémité 110a de la fibre 110 et présente un diamètre externe d1 sensiblement égal au diamètre courant D1 de cette fibre. Il maintient la brosse 112 sur toute la longueur L' de cette dernière. En effet, l'organe 120 dépasse au-delà de l'extrémité 110a de la fibre sur une longueur supérieure à celle de la brosse. De même, l'organe de maintien 220 de la figure 2, maintient la brosse 212 sur toute sa longueur L".

L'organe de maintien 120 ou 220 est constitué par un fil enroulé en hélice, dont les spires 122 ou 222 délimitent un canal 124 ou 224, apte à recevoir la tige de la brosse. Sur au moins un tronçon de cet organe de maintien, tronçon dont la longueur est au moins égale à celle de la brosse, les spires sont espacées axialement les unes des autres de manière à permettre le passage des poils de la brosse.

En fait, les poils de la brosse sont de préférence disposés en hélice et, sur chaque spire de cette hélice, regroupés par groupes de trois ou quatre poils ou davantage. L'espacement "e" entre les spires de l'organe de maintien permet le passage d'une spire entière des poils de la brosse, et est donc de l'ordre de trois à vingt fois l'épaisseur d'un poil.

Comme on le voit sur les figures 1 et 2, dans le tronçon de l'organe de maintien sur lesquels les spires sont écartées, le pas de l'hélice de cet organe est sensiblement égal au pas de l'hélice que forment les poils de la brosse. On comprend donc, comme on le précisera dans la suite, que la brosse et l'organe de maintien peuvent être mis en place l'une par rapport à l'autre par un mouvement de vissage.

Sur la figure 1, l'extrémité 110a de la fibre 110 est munie d'un manchon de raccordement 126 susceptible de coopérer avec la première extrémité 120a de l'organe de maintien 120 pour raccorder ce dernier à la fibre 110. Ce manchon est sensiblement cylindrique et son rayon courant est inférieur au rayon courant de la fibre. L'écart entre ces rayons est sensiblement égal à l'épaisseur du fil qui constitue l'organe de maintien. On s'assure ainsi, même dans la région de raccordement de la fibre et de l'organe de maintien, que le diamètre externe de cet organe est inférieur ou sensiblement égal au diamètre courant de la fibre. En fait, le fil qui constitue l'organe de maintien peut être métallique et il est préférable qu'il ne dépasse pas au-delà des dimensions diamétrales de la fibre pour éviter de rayer le conduit que l'on nettoie. On peut en revanche sans dommage, dans la mesure où la fibre est lisse, faire en sorte que le diamètre externe de l'organe de maintien soit légèrement inférieur au diamètre de la fibre.

A cet égard, il faut noter que, sur le tronçon qui supporte la brosse, les poils de cette dernière évitent tout contact direct entre l'organe de maintien et le conduit à nettoyer.

Sur la figure 1, la tige 114 de la brosse est fixée à la première extrémité 110a de la fibre. L'extrémité de cette tige peut en effet être engagée dans un court alésage axial de la fibre et être collée dans ce dernier. Elle passe alors évidemment à travers un alésage axial du manchon 126. La brosse est donc constamment solidaire de la fibre 110.

Entre deux nettoyages, il importe de décontaminer et de nettoyer le dispositif. A cette occasion, il est bien sûr préférable de pouvoir totalement accéder aux poils de la brosse. Pour ce faire, l'organe de maintien 120 de la figure 1 est démontable et il peut être monté ou démonté à la première extrémité de la fibre 110 par un mouvement de vissage par rapport à la brosse. Dans le sens du montage, le mouvement de vissage se continue jusqu'à ce que l'extrémité 120a de l'organe de maintien coopère avec le manchon 126.

L'organe de maintien 120 est fixé axialement par vissage sur la brosse 112.

On voit sur la figure 1 que le manchon est constitué par un élément rapporté et fixé à l'extrémité 110a de la fibre. Le manchon peut en effet être constitué par une pièce rigide, par exemple en métal, qui en maintenant rigidement les spires d'extrémité de l'organe de maintien permet d'initier la courbure de ce dernier lorsque le dispositif est inséré dans des courbes des conduits à nettoyer. Il importe en effet d'éviter que l'organe de maintien ne se retrouve légèrement décalé radialement par rapport à la fibre pour éviter qu'il ne vienne au contact des parois internes du conduit à nettoyer.

La première extrémité 110a de la fibre 110 présente une cavité cylindrique 111. Le manchon 126 présente une première partie 126a logée dans cette cavité 111 et fixée à ses parois. Le manchon présente une deuxième partie 126b qui dépasse au-delà de la première extrémité 110a de la fibre. C'est cette deuxième partie qui coopère avec l'extrémité 120a de l'organe de maintien. Le diamètre externe de la deuxième partie 126b est inférieur ou égal au diamètre interne de l'organe de maintien 120. Cette deuxième partie sert à guider l'organe de maintien lors de son vissage et surtout, lorsque cet organe est en place, à éviter qu'il ne se déplace transversalement à l'axe de la tige 114. Comme on l'a évoqué précédemment, le manchon présente un perçage axial de dimension adaptée à celle de la tige de la brosse.

L'embout 118 présente une première portion 118a de diamètre adapté à celui du canal délimité par les spires de l'organe de maintien 120 et une deuxième partie 118b qui présente une extrémité arrondie. Ces deux parties sont raccordées l'une à l'autre par un épaulement 118c susceptible de coopérer avec l'extrémité libre 120b de l'organe de maintien. En fait, dans le mode de réalisation de la figure 1, l'embout peut être définitivement fixé, par exemple par collage, à l'organe de maintien 120, ou être vissé dans ce dernier et, dans ce cas, présenter un filetage adapté.

La deuxième partie 118b de l'embout, dont le diamètre est supérieur au diamètre externe de l'organe de maintien 120, permet d'éviter tout contact entre cet organe et le conduit à nettoyer.

La figure 1bis montre une variante de montage, utilisant un manchon cylindrique creux 126'. Dans cette variante, l'extrémité 110'a de la fibre 110' présente un épaulement et une portion extrême de diamètre réduit 111'. Le manchon 126' est emmanché sur cette portion 111' et y est fixé. Son diamètre externe est tout au plus égal à celui de la fibre 110'.

Dans cette position, la cavité cylindrique 127' ménagée entre l'extrémité de la fibre et l'extrémité libre du manchon 126' sert de logement à la première extrémité 120'a de l'organe de maintien 120'. Cette conformation permet d'éviter le débattement de cette première extrémité 120'a par rapport à l'axe de la tige 114' de la brosse 112' sur laquelle l'organe de maintien est vissé.

Dans le mode de réalisation de la figure 2, sur laquelle les éléments communs à la figure 1 sont affectés des mêmes références, augmentées de 100, la première extrémité 220a de l'organe de maintien est fixée au manchon 226. La brosse 212 est alors susceptible d'être mise en place dans ledit organe de maintien par un mouvement de vissage et d'en être dégagé par un mouvement de dévissage. Elle est maintenue axialement dans l'organe de maintien grâce au fait que ses poils passent entre les spires de ce dernier. A l'instar du manchon 126, le manchon 226 est sensiblement cylindrique et présente un rayon courant inférieur au rayon courant de la fibre 210, l'écart entre ces rayons étant sensiblement égal à l'épaisseur du fil qui constitue l'organe de maintien.

Dans l'exemple représenté, le manchon 226 est simplement constitué par une portion extrême de diamètre réduit de l'extrémité 210a de la fibre. On peut évidemment de la même façon utiliser un manchon analogue au manchon 126 précédemment décrit. Il n'est cependant pas nécessaire qu'il présente un perçage axial dans la mesure où la tige de la brosse n'y est pas engagée.

L'extrémité 220a de l'organe de maintien est fixée à ce manchon 226 par tout moyen approprié, tel que le collage ou le vissage à force. Dans ce dernier cas, le manchon 226 peut présenter un filetage.

L'embout de protection 218 présente une première portion 218a, pouvant être fixée à la tige 214 de la brosse, de diamètre adapté à celui du canal délimité par les spires de l'organe de maintien et raccordée à l'extrémité arrondie 218b par un épaulement 218c susceptible de coopérer avec l'extrémité libre de l'organe de maintien. La brosse 212 est donc correctement mise en place lorsque l'épaulement 218c vient en butée avec la première spire de l'extrémité libre 220b de l'organe de maintien.

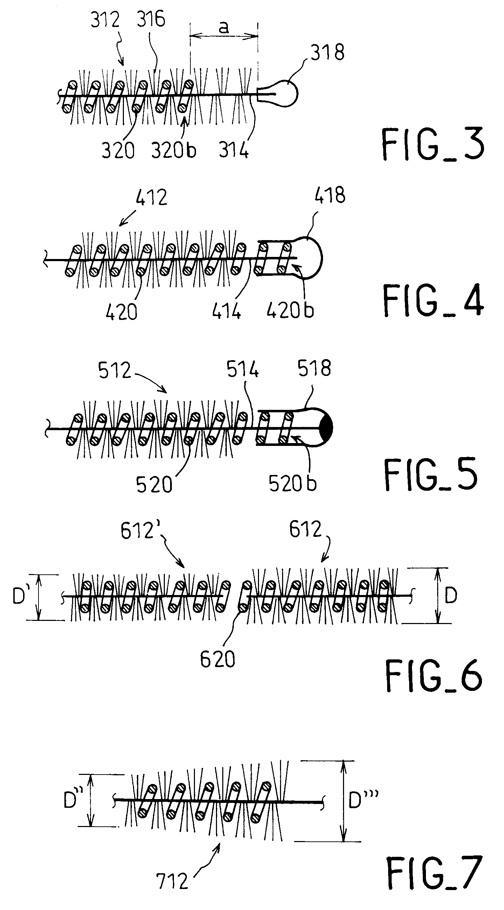

Les figures 3 à 5 montrent des variantes pour lesquelles les embouts peuvent être métalliques. Pour alléger ces figures, seules les régions d'extrémité des brosses sont représentées. Les organes de maintien, de même que les brosses, sont de configurations analogues à celles précédemment décrites.

Sur la figure 3, l'embout 318 est directement fixé à l'extrémité libre de la tige 314 de la brosse 312, par tout moyen approprié tel que sertissage, collage ou vissage. La brosse dépasse donc sur une légère distance "a" au-delà de l'extrémité libre 320b de l'organe de maintien 320. Il faut toutefois noter que cette extrémité libre n'est pas agressive puisqu'elle est noyée dans les poils 316 de la brosse. De plus, la distance "a" est suffisamment faible pour que l'absence de maintien à l'extrémité de la brosse ne nuise ni à son efficacité, ni à la solidité de la tige. Le diamètre de l'embout 318 peut être inférieur à celui de l'organe de maintien.

Sur la figure 4, l'embout 418 coiffe l'extrémité libre 420b de l'organe de maintien 420 et peut être rétreint sur cette dernière pour y être définitivement fixé. La tige 414 de la brosse s'étend avantageusement jusqu'à l'intérieur de l'embout.

Sur la figure 5, l'embout 518 coiffe également extrémité libre 520b de l'organe de maintien 520. Cependant, cet embout est directement fixé à l'extrémité de la tige 514 de la brosse 512 par collage, soudure ou tout autre moyen analogue. L'ensemble constitué par la brosse 512 et l'embout 518 peut ainsi être monté amovible par rapport à l'organe de maintien 520.

Les embouts 318, 418 et 518 sont arrondis pour éviter d'endommager les conduits nettoyés à l'aide du dispositif.

La figure 6 montre deux brosses 612 et 612' successivement disposées dans l'organe de maintien 620. Ces deux brosses peuvent donc être disposées l'une à la suite de l'autre à la première extrémité de la fibre. Elles présentent des diamètres D et D' différents. Elles peuvent être séparées comme le montre la figure, ou réunies par la même tige.

On a en effet constaté que le nettoyage d'un conduit est plus efficace lorsque le diamètre des poils de la brosse utilisée est très légèrement supérieur au diamètre interne de ce conduit. Il se trouve que le diamètre des conduits des instruments médicaux d'investigation ou de prélèvement interne varie selon le type d'examen ou d'opération que l'on réalise avec ces instruments, la morphologie ou encore l'age du patient. Le dispositif équipé de plusieurs brosses de diamètre différent permet donc de nettoyer, avec la même efficacité, différents instruments. Selon le diamètre du conduit nettoyé, l'une des brosses sera davantage mise à contribution, tandis que les poils de la ou des autres pourront se rabattre axialement ou ne pas entrer en contact avec le conduit à nettoyer.

La figure 7 montre une brosse 712 dont le diamètre varie sur sa longueur et augmente entre un petit diamètre D" et un grand diamètre D"'. Cette augmentation peut être progressive comme sur la figure, auquel cas la brosse est tronconique. Elle peut également se faire graduellement ou par à-coups, la brosse pouvant même présenter des portions cylindriques et des portions tronconiques. Cette conformation permet d'assurer qu'il existe toujours une région de la brosse dans laquelle le diamètre des poils permet un nettoyage très efficace du conduit.

La brosse 712 peut être prévue seule ou associée à une ou plusieurs autres brosses telles que les brosses cylindriques 612 et 612'.

La fibre 110 de la figure 1 présente, au voisinage de sa deuxième extrémité 110b, une section amincie 110c dont la flexibilité est supérieure à celle de sa partie courante. De préférence, cette section est progressivement amincie, ce qui permet de faire varier progressivement la flexibilité de la fibre dans cette région. C'est également le cas de la fibre 210. Dans certains cas, par exemple pour le nettoyage de conduits présentant localement de très fortes courbures, il est en effet préférable d'insérer d'abord le dispositif par la deuxième extrémité 110b ou 210b de la fibre. La section amincie, de grande flexibilité, pourra passer les coudes sans difficulté et permettra donc d'insérer totalement le dispositif.

On constate sur la figure 2 que la fibre 210 présente une autre portion amincie 210d située au voisinage de sa première extrémité 210a. Cette portion 210d confère une flexibilité locale importante juste avant la brosse 212.

On peut doter le dispositif d'une deuxième brosse située à la deuxième extrémité de la fibre. Cette deuxième brosse, qui peut être équipée d'un organe de maintien analogue à celui qui équipe la première, est avantageusement plus courte et plus large que la première. Dans ce cas, c'est d'abord la deuxième brosse que l'on insère dans le conduit à nettoyer.

## Revendications

1. Dispositif de nettoyage de conduits d'instruments médicaux d'investigation ou de prélèvement interne, comprenant un élément cylindrique allongé (110, 210) étant constitue par une fibre et ayant une surface externe lisse et une brosse (112, 212), prévue à une première extrémité (110a, 210a) dudit élément, montée sur une tige longitudinale (114, 214) et présentant des poils sensiblement radiaux (116, 216),
caractérisé en ce qu'il comporte, en outre, un organe de maintien (120, 220) de la brosse (112, 212), monté à la première extrémité (110a, 210a) de la fibre (110, 210), de diamètre externe (d1) sensiblement égal à celui de ladite fibre et susceptible de maintenir la brosse sur sensiblement toute la longueur (L', L'') de cette dernière, ledit organe étant constitué par un fil enroulé en hélice, dont les spires (122, 222) délimitent un canal (124, 224) apte à recevoir la tige (114, 214) de la brosse, et présentant un tronçon, de longueur sensiblement égale à celle de la brosse, sur lequel les spires sont espacées axialement de manière à permettre le passage des poils (116, 216) de la brosse.

2. Dispositif selon la revendication 1, caractérisé en ce que la première extrémité (110a, 210a) de la fibre (110, 210) est munie d'un manchon de raccordement (126, 226) susceptible de coopérer avec une première extrémité (120a, 220a) de l'organe de maintien (120, 220) pour raccorder ce dernier à la fibre, ledit manchon étant sensiblement cylindrique et présentant un rayon courant inférieur au rayon courant de la fibre, l'écart entre lesdits rayons étant sensiblement égal à l'épaisseur du fil constituant l'organe de maintien (120, 220).

3. Dispositif selon la revendication 1, caractérisé en ce que la première extrémité (110'a) de la fibre (110') présente une portion (111') de diamètre réduit et est munie d'un manchon cylindrique creux (126') emmanché sur ladite portion (111'), et en ce que la cavité cylindrique (127') ménagée entre l'extrémité (110'a) de la fibre et l'extrémité libre du manchon sert de logement à la première extrémité (120'a) de l'organe de maintien (120').

4. Dispositif selon l'une quelconque des revendications 1 à 3, caractérisé en ce que la tige (114, 114') de la brosse (112, 112') est fixée à la première extrémité (110a, 110'a) de la fibre (110, 110'), et en ce que l'organe de maintien (120, 120') est susceptible d'être monté à la première extrémité de la fibre (110, 110') par un mouvement de vissage par rapport à la brosse (112, 112').

5. Dispositif selon l'une quelconque des revendications 3 et 4, caractérisé en ce que la première extrémité (110a) de la fibre (110) présente une cavité cylindrique (111), et en ce que le manchon de raccordement (126) présente une première partie (126a), logée dans ladite cavité (111) et fixée aux parois de cette dernière, une deuxième partie (126b), faisant saillie au-delà de la première extrémité (110a) de la fibre (110) et susceptible de coopérer avec la première extrémité (120a) de l'organe de maintien (120), ainsi qu'un perçage axial de dimensions adaptées à celles de la tige (114) de la brosse.

6. Dispositif selon l'une quelconque des revendications 1 et 2, caractérisé en ce que la première extrémité de l'organe de maintien (220a) est fixée au manchon (226), et en ce que la brosse (212) est susceptible d'être mise en place dans ledit organe de maintien (220) par un mouvement de vissage.

7. Dispositif selon l'une quelconque des revendications 1 à 6, caractérisé en ce que la longueur (L, L', L'') de la brosse est supérieure au triple du diamètre (D, D') de ladite brosse.

8. Dispositif selon l'une quelconque des revendications 1 à 7, caractérisé en ce que le diamètre (D'', D''') de la brosse (712) varie sur la longueur de cette dernière.

9. Dispositif selon l'une quelconque des revendications 1 à 8, caractérisé en ce qu'il comporte au moins deux brosses (612, 612') disposées l'une à la suite de l'autre à la première extrémité de la fibre (110, 110', 210), et présentant des diamètres (D, D') différents.

10. Dispositif selon l'une quelconque des revendications 1 à 9, caractérisé en ce que la fibre (110, 210) présente, au voisinage d'au moins l'une de ses extrémités (110b, 210b ; 210a), une section progressivement amincie (110c, 210c ; 210d), dont la flexibilité est supérieure à celle de la partie courante de ladite fibre.

11. Dispositif selon l'une quelconque des revendications 1 à 10, caractérisé en ce qu'il comporte un embout (118, 218, 318, 418, 518) monté à l'extrémité du dispositif opposée à la deuxième extrémité de la fibre.

12. Dispositif selon la revendication 11, caractérisé en ce que l'embout (118, 418) est fixé à l'extrémité libre (120b, 420b) de l'organe de maintien (120, 420).

13. Dispositif selon la revendication 11, caractérisé en ce que l'embout (218, 318, 518) est fixé à l'extrémité libre de la tige (214, 314, 514) de la brosse.

14. Dispositif selon l'une quelconque des revendications 11 à 13, caractérisé en ce que l'embout (418, 518) coiffe l'extrémité libre (420b, 520b) de l'organe de maintien (420, 520).

15. Dispositif selon l'une quelconque des revendications 11 et 12, caractérisé en ce que l'embout (118, 218) présente une première portion (118a, 218a), de diamètre adapté à celui du canal délimité par les spires de l'organe de maintien (120, 220) et raccordée à l'extrémité arrondie (118b, 218b) dudit embout par un épaulement (118c, 218c) susceptible de coopérer avec l'extrémité libre (120b, 220b) de l'organe de maintien (120, 220).

16. Dispositif selon l'une quelconque des revendications 1 à 15, caractérisé en ce qu'il comporte une deuxième brosse disposée à la deuxième extrémité de la fibre

## Claims

1. A device for cleaning the ducts of medical instruments for internal investigation or sample-taking, the device comprising an elongate cylindrical element (110, 210) constituted by a rod and having a smooth outside surface and a brush (112, 212) provided at a first end (110a, 210a) of said element, the brush being mounted on a longitudinal spine (114, 214) and having substantially radial bristles (116, 216),
characterised in that the device further includes a support member (120, 220) for supporting the brush (112, 212), mounted at the first end (110a, 210a) of the rod (110, 210), the outside diameter (d1) of the support member being substantially equal to that of the rod, and the support member being suitable for supporting the brush substantially over the entire length (L', L'') thereof, said member being constituted by a helically wound wire whose turns (122, 222) define a channel (124, 224) suitable for receiving the spine (114, 214) of the brush, and having a length substantially equal to the length of the brush over which length the turns are spaced apart axially so as to allow the bristles (116, 216) of the brush to pass between them.

2. A device according to claim 1, characterised in that the first end (110a, 210a) of the rod (110, 210) is provided with a coupling sleeve (126, 226) suitable for co-operating with a first end (120a, 220a) of the support member (120, 220) for coupling it to the rod, said sleeve being substantially cylindrical and having a running radius that is smaller than the running radius of the rod, the difference between said radii being substantially equal to the thickness of the wire constituting the support member (120, 220).

3. A device according to claim 1, characterised in that the first end (110'a) of the rod (110') has a portion (111') of smaller diameter and is provided with a hollow cylindrical sleeve (126') fitted on said portion (111'), and in that the cylindrical cavity (127') provided between the end (110'a) of the rod and the free end of the sleeve serves as a housing for the first end (120'a) of the support member (120').

4. A device according to any one of claims 1 to 3, characterised in that the spine (114, 114') of the brush (112, 112') is fixed to the first end (110a, 110'a) of the rod (110, 110'), and in that the support member (120, 120') is suitable for being mounted at the first end of the rod (110, 110') by screwing motion relative to the brush (112, 112').

5. A device according to claim 3 or 4, characterised in that the first end (110a) of the rod (110) has a cylindrical cavity (111), and in that the coupling sleeve (126) has a first portion (126a) housed in said cavity (111) and fixed to the walls thereof, a second portion (126b) projecting beyond the first end (110a) of the rod (110) and suitable for cooperating with the first end (120a) of the support member (120), and an axial bore of dimensions adapted to those of the spine (114) of the brush.

6. A device according to claim 1 or 2, characterised in that the first end of the support member (220a) is fixed to the sleeve (126), and in that the brush (212) is suitable for being installed in said support member (220) by screwing motion.

7. A device according to any one of claims 1 to 6, characterised in that the length (L, L', L'') of the brush is greater than three times the diameter (D, D') of said brush.

8. A device according to any one of claims 1 to 7, characterised in that the diameter (D'' D''') of the brush (712) varies along its length.

9. A device according to any one of claims 1 to 8, characterised in that it includes at least two brushes (612, 612') disposed one after the other at the first end of the rod (110, 110', 210), and having different diameters (D, D').

10. A device according to any one of claims 1 to 9, characterised in that the rod (110, 210) has, in the vicinity of at least one of its ends (110b, 210b; 210a), a section that is progressively reduced (110c, 210c; 210d) of greater flexibility than the running portion of said rod.

11. A device according to any one of claims 1 to 10, characterised in that it includes an endpiece (118, 218, 318, 418, 518) mounted at the end of the device opposite the second end of the rod.

12. A device according to claim 11, characterised in that the endpiece (118, 418) is fixed to the free end (120b, 420b) of the support member (120, 420).

13. A device according to claim 11, characterised in that the endpiece (218, 318, 518) is fixed to the free end of the spine (214, 314, 514) of the brush.

14. A device according to any one of claims 11 to 13, characterised in that the endpiece (418, 518) is fitted over the free end (420b, 520b) of the support member (420, 520).

15. A device according to claim 11 or 12, characterised in that the endpiece (118, 218) has a first portion (118a, 218a) of a diameter that matches that of the channel defined by the turns of the support member (120, 220) and united to the rounded end (118b, 218b) of said endpiece by a shoulder (118c, 218c) suitable for co-operating with the free end (120b, 220b) of the support member (120, 220).

16. A device according to any one of claims 1 to 15, characterised in that it includes a second brush disposed at the second end of the rod.

## Patentansprüche

1. Vorrichtung zur Reinigung von Kanälen von medizinischen Geräten für interne Untersuchungen oder Entnahmen, mit einem aus einem Fiberelement bestehenden, zylindrischen Längselement (110, 210), das eine glatte Außenfläche und eine an einem ersten Ende (110a, 210a) des Elements vorgesehene Bürste (112, 212) aufweist, die an einem Längsschaft (114, 214) angebracht ist und im Wesentlichen radiale Haare (116, 216) aufweist, dadurch gekennzeichnet, dass sie weiters ein an dem ersten Ende (110a, 210a) des Fiberelements (110, 210) angebrachtes Halteorgan (120, 220) für die Bürste (112, 212) mit einem äußeren Durchmesser (d1), im Wesentlichen gleich dem des Fiberelements, das dazu geeignet ist, die Bürste im Wesentlichen auf deren gesamter Länge (L', L'') zu halten, wobei das Organ durch einen schraubenlinienförmig gewickelten Faden gebildet ist, dessen Windungen (122, 222) einen Kanal (124, 224) begrenzen, der den Schaft (114, 214) der Bürste aufnehmen kann, und einen Teilabschnitt mit einer im Wesentlichen der Länge der Bürste entsprechenden Länge aufweist, auf dem die Windungen mit axialen Abständen angeordnet sind, um den Durchtritt der Haare (116, 216) der Bürste zu erlauben.

2. Vorrichtung nach Anspruch 1, dadurch gekennzeichnet, dass das erste Ende (110a, 210a) des Fiberelements (110, 210) einen Verbindungsstutzen (126, 226) aufweist, der dazu geeignet ist, mit einem ersten Ende (120a, 220a) des Halteorgans (120, 220) zu dessen Verbindung mit dem Fiberelement zusammenzuarbeiten, wobei der Stutzen im Wesentlichen zylindrisch ist und einen laufenden Radius aufweist, der kleiner ist als der laufende Radius des Fiberelements, wobei der Unterschied zwischen diesen Radien im Wesentlichen der Dicke des das Halteorgan (120, 220) bildenden Fadens entspricht.

3. Vorrichtung nach Anspruch 1, dadurch gekennzeichnet, dass das erste Ende (110'a) des Fiberelements (110') einen Abschnitt (111') mit verringertem Durchmesser aufweist und mit einem hohlen zylindrischen, auf den Abschnitt (111') aufgeschrumpften Stutzen (126') versehen ist, und dass der zwischen dem Ende (110'a) des Fiberelements und dem freien Ende des Stutzens angebrachte zylindrische Hohlraum (127') als Sitz für das erste Ende (120'a) des Halteorgans (120') dient.

4. Vorrichtung nach einem der Ansprüche 1 bis 3, dadurch gekennzeichnet, dass der Schaft (114, 114') der Bürste (112, 112') an dem ersten Ende (110a, 110'a) des Fiberelements (110, 110') befestigt ist, und dass das Halteorgan (120, 120') durch eine Schraubbewegung bezüglich der Bürste (112, 112') auf dem ersten Ende des Fiberelements (110, 110') anbringbar ist.

5. Vorrichtung nach Anspruch 3 oder 4, dadurch gekennzeichnet, dass das erste Ende (110a) des Fiberelements (110) einen zylindrischen Hohlraum (111) aufweist, und dass der Verbindungsstutzen (126) einen im Hohlraum (111) gelagerten und an der Wand des letzteren befestigten ersten Abschnitt (126a), einen über das erste Ende (110a) des Fiberelements (110) hinausragenden und mit dem ersten Ende (120a) des Halteorgans (120) zusammenarbeitenden zweiten Abschnitt (126b) sowie eine axiale Bohrung mit an die des Schaftes (114) der Bürste angepassten Abmessungen aufweist.

6. Vorrichtung nach Anspruch 1 oder 2, dadurch gekennzeichnet, dass das erste Ende des Halteorgans (220a) an dem Stutzen (226) befestigt ist, und dass die Bürste (212) durch eine Schraubbewegung im Halteorgan (220) anbringbar ist.

7. Vorrichtung nach einem der Ansprüche 1 bis 6, dadurch gekennzeichnet, dass die Länge (L, L', L'') der Bürste größer ist als der dreifache Durchmesser (D, D') der Bürste.

8. Vorrichtung nach einem der Ansprüche 1 bis 7, dadurch gekennzeichnet, dass der Durchmesser (D'', D''') der Bürste (712) auf deren Länge variiert.

9. Vorrichtung nach einem der Ansprüche 1 bis 8, dadurch gekennzeichnet, dass sie zumindest zwei hintereinander an dem ersten Ende des Fiberelements (110, 110', 210) angeordnete und verschiedene Durchmesser (D, D') aufweisende Bürsten (612, 612') aufweist.

10. Vorrichtung nach einem der Ansprüche 1 bis 9, dadurch gekennzeichnet, dass das Fiberelement (110, 210) benachbart zumindest einem seiner Enden (110b, 210b; 210a) einen allmählich verjüngten Abschnitt (110c, 210c, 210d) aufweist, dessen Flexibilität größer als jene des übrigen Abschnitts des Fiberelements ist.

11. Vorrichtung nach einem der Ansprüche 1 bis 10, dadurch gekennzeichnet, dass sie einen an einem dem zweiten Ende des Fiberelements gegenüberliegenden Ende der Vorrichtung angebrachten Ansatz (118, 218, 318, 418, 518) aufweist.

12. Vorrichtung nach Anspruch 11, dadurch gekennzeichnet, dass der Ansatz (118, 418) am freien Ende (120b, 420b) des Halteorgans (120, 420) befestigt ist.

13. Vorrichtung nach Anspruch 11, dadurch gekennzeichnet, dass der Ansatz (218, 318, 518) am freien Ende des Schaftes (214, 314, 514) der Bürste befestigt ist.

14. Vorrichtung nach einem der Ansprüche 11 bis 13, dadurch gekennzeichnet, dass der Ansatz (418, 518) das freie Ende (420b, 520b) des Halteorgans (420, 520) bedeckt.

15. Vorrichtung nach einem der Ansprüche 11 und 12, dadurch gekennzeichnet, dass der Ansatz (118, 218) einen ersten Abschnitt (118a, 218a) aufweist, dessen Durchmesser dem des durch die Windungen des Halteorgans (120, 220) begrenzten Kanals entspricht, und der mit einem abgerundeten Ende (118b, 218b) des Ansatzes über eine Schulter (118c, 218c) verbunden ist, die dazu geeignet ist, mit dem freien Ende (120b, 220b) des Halteorgans (120, 220) zusammenzuarbeiten.

16. Vorrichtung nach einem der Ansprüche 1 bis 15, dadurch gekennzeichnet, dass sie eine zweite, an dem zweiten Ende des Fiberelements angebrachte Bürste aufweist.
